# EUROPEAN PATENT APPLICATION

(11) **EP 4 218 428 A1**
(43) Date of publication of application: **02.08.2023**
(21) Application number: 21898458.1
(22) Date of filing: 15.11.2021
(51) Int. Cl.: A23L 27/30, A23L 29/00

(54) **COMPOSITION HAVING IMPROVED SWEETNESS COMPRISING GLUCOSE-TRANSFERRED STEVIA**

(30) Priority: 25.11.2020 KR 20200160304
(71) Applicant: CJ Cheiljedang Corporation, Seoul 04560 (KR)
(72) Inventor: KIM, Jungeun, Seoul 04560 (KR); PARK, Sunghee, Seoul 04560 (KR); CHU, Sun, Seoul 04560 (KR); BYUN, Sung Bae, Seoul 04560 (KR); JU, Jae Yeong, Seoul 04560 (KR)
(74) Representative: Regimbeau
(86) International application number: PCT/KR2021/016616
(87) International publication number: WO 2022/114632

(57) **Abstract**

Provided is a sweetener composition including glucosylated rebaudioside A, glucosylated stevioside, and glucosylated rebaudioside C, a composition for preparing a sweetener, a method of preparing a sweetener using a steviol glycoside by-product, and a method of improving sweetness quality.

## Description

### [Technical Field]

The present disclosure relates to a sweetener composition including glucosylated rebaudioside A, glucosylated stevioside, and glucosylated rebaudioside C, a composition for preparing a sweetener, a method of preparing a sweetener using a steviol glycoside by-product, and a method of improving sweetness quality.

### [Background Art]

In response to concerns about diseases caused by excessive sugar intake of modern people and recommendations by the World Health Organization (WHO) on lowering sugar consumption, policies to reduce sugar intake are actively being discussed in developed countries, and sugar substitutes have been continuously developed in the market. In this regard, stevia, which has recently received considerable attention as a sugar substitute, is a natural high-intensity sweetener having 200 to 300 times the sweetness of sugar, and has been reported to have zero calories and cause no side effects in the human body, and thus the potential thereof as a sugar substitute has been approved.

Among commercially available stevia sweeteners, high-purity rebaudioside A with a purity of 95% to 97% is known to have the highest commercial value. However, a crystallization process is required in order to prepare high-purity stevia, a high-intensity sweetener, and components having low sweetness quality are contained in large quantities in a liquid-phase by-product generated while a solid is separated. Components of the by-product are typically stevioside, rebaudioside C, and the like. Since the commercial value of the by-product is low due to strong bitter taste, the by-product is dried to be used as inexpensive fertilizer or animal feed, or it is sold at a low value.

In order to solve such problems, attempts have been made to artificially increase the content of rebaudioside A and to produce rebaudioside A in a large quantity by breeding a plant including a high rebaudioside A content (U.S. Patent Application Publication No. 2009-0214753). However, since these methods cannot provide sufficient efficiency and have limitations in terms of costs and quality, there is a need for a method of reducing production of the by-product by increasing the production efficiency of stevia sweeteners, or a method of increasing the commercial value of the by-product.

### [Disclosure]

### [Technical Problem]

The present inventors have found that a glucosylated stevia composition having a certain composition ratio may be prepared by applying a culture broth of *Lactobacillus mali,* as a crude enzyme liquid, to a by-product generated in a steviol production process and have confirmed that the glucosylated stevia composition having the certain composition ratio may be used as a high-value-added product with improved sweetness quality.

### [Technical Solution]

An object of the present disclosure is to provide a sweetener composition including glucosylated rebaudioside A, glucosylated stevioside, and glucosylated rebaudioside C.

Another object of the present disclosure is to provide a composition for preparing a sweetener including a steviol glycoside by-product.

Another object of the present disclosure is to provide a method of preparing a sweetener composition including glucosylated rebaudioside A, glucosylated stevioside, and glucosylated rebaudioside C.

Another object of the present disclosure is to provide a method of improving sweetness quality.

Another object of the present disclosure is to provide a use for preparing a sweetener composition.

Another object of the present disclosure is to provide a use for improving sweetness quality.

### [Advantageous Effects]

In the case of using the method of improving sweetness quality and the method of preparing a sweetener, a sweetener composition having a certain composition ratio may be prepared using a by-product generated in a steviol production process. In addition, since the sweetener composition having the certain composition ratio and the composition for preparing the sweetener have excellent sweetness quality, they may be used for manufacturing high-value-added products such as foods and feeds in various industrial fields.

### [Brief Description of Drawings]

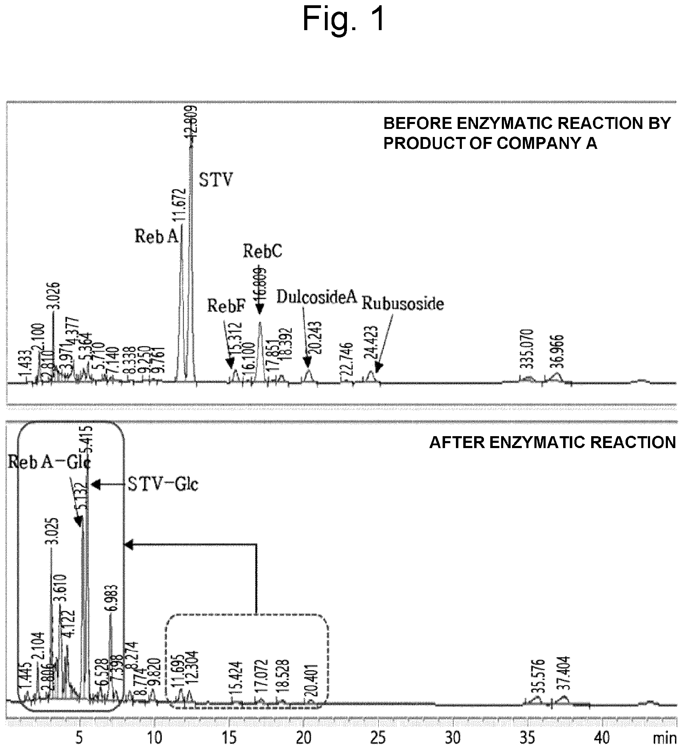

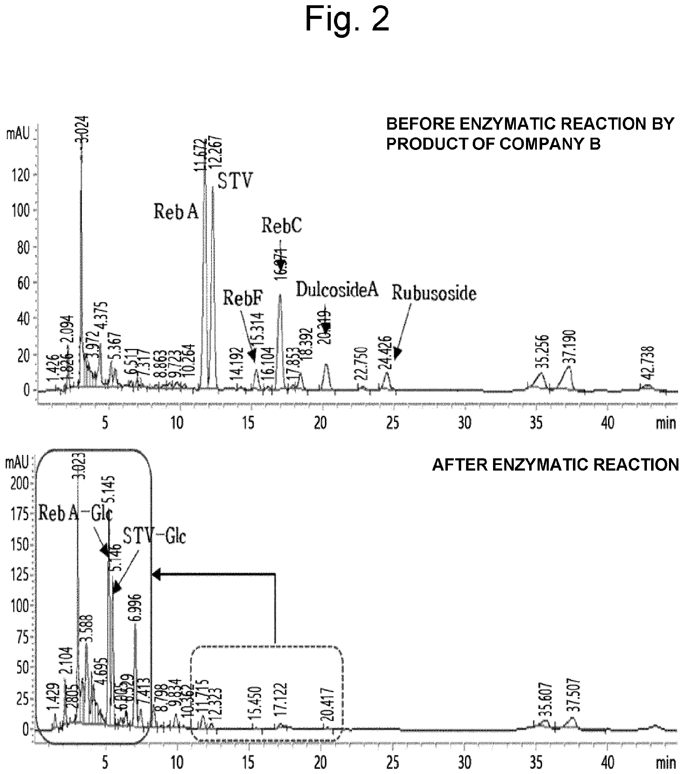

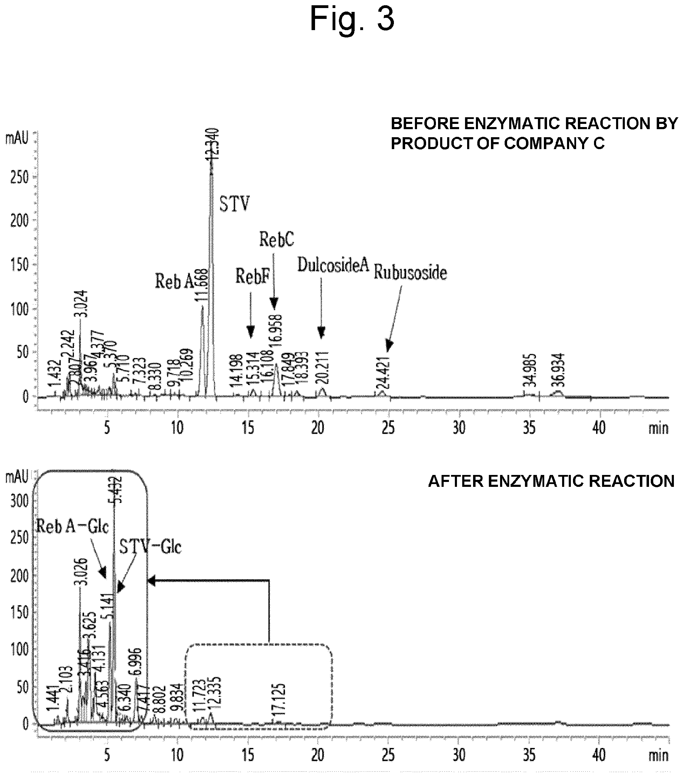

FIGS. 1 to 3 show HPLC results of compositions including glucosylated steviol glycosides prepared from steviol glycoside by-products.

### [Best Mode]

Hereinafter, the present disclosure will be described in detail. Meanwhile, in the present disclosure, each of the explanations and exemplary embodiments disclosed herein can be applied to other explanations and exemplary embodiments. Also, in the present disclosure, all combinations of various factors disclosed herein belong to the scope of the present disclosure. Furthermore, the scope of the present disclosure should not be limited by the specific disclosure provided hereinbelow.

An aspect of the present disclosure provides a sweetener composition including glucosylated rebaudioside A, glucosylated stevioside, and glucosylated rebaudioside C. Specifically, the sweetener composition includes 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener composition.

The sweetener composition may have improved sweetness quality compared to commercially available high-purity rebaudioside A, specifically, the sweetener composition may have improved sweetness quality compared to rebaudioside A having a purity of 95% or more, and more specifically, improvement of the sweetness quality may include improved sweetness, reduced off-flavor, and/or reduced off-odor, without being limited thereto.

The sweetener composition may have a certain composition ratio via a one-pot reaction by treating a typical steviol glycoside by-product with transglucosylase including an amino acid sequence of SEQ ID NO: 1, a microorganism expressing the same, or a culture of the microorganism or *Lactobacillus mali,* without being limited thereto.

The sweetener composition may further include a cofactor capable of improving stability of the sweetener composition, without being limited thereto. Examples of the cofactor may include a metal ion, a metal salt, an excipient, and a preservative, without being limited thereto.

The glucosylated rebaudioside A, glucosylated stevioside, and glucosylated rebaudioside C are glucosylated steviol glycosides in which glucose is bound to rebaudioside A, stevioside, and rebaudioside C, which are steviol glycosides, respectively.

In the present disclosure, "steviol glycoside" is a natural sweetener and may be represented by Formula 1 below.

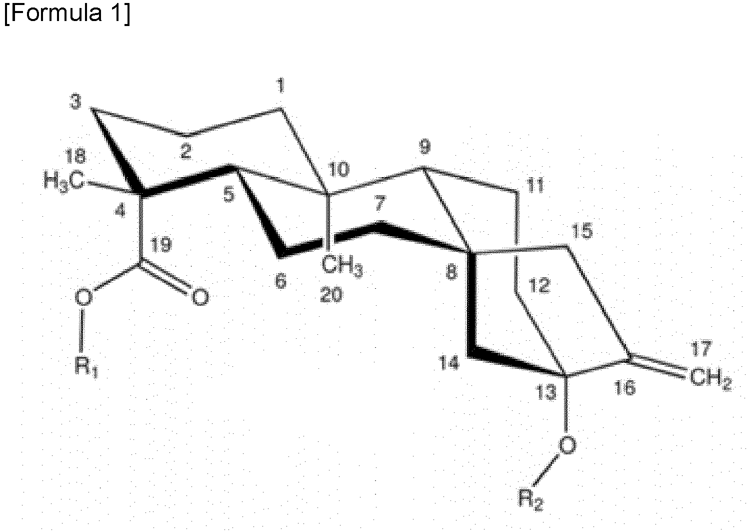

In Formula 1, at R₁, hydrogen (H) may be bound or 1 to 3 glucose molecules may be bound via a β bond, and at R₂, one molecule of glucose, xylose, and rhamnose may be bound via a β bond and 0 to 2 glucose molecules may be bound thereto via a β bound, without being limited thereto.

The steviol glycoside may include at least one selected from the group consisting of stevioside, rubusoside, dulcoside A, rebaudioside A, rebaudioside C, rebaudioside D, rebaudioside E, rebaudioside F, and rebaudioside M, without being limited thereto.

These steviol glycosides may be modified into glucosylated steviol glycosides in the presence of a crude enzyme liquid having a transglucosylating activity, the transglucosylase, transglucosylase including an amino acid sequence of SEQ ID NO: 1, a microorganism expressing the same, or a culture of the microorganism or *Lactobacillus mali,* and a glucose donor, without being limited thereto.

In the present disclosure, the "glucose donor" refers to any of oligomers or polymers of glucose and cyclic forms thereof participating in reactions to transfer one or more glucose molecules to a steviol glycoside in the presence of transglucosylase, and specifically may be sugar, but is not limited thereto.

In the present disclosure, since "rebaudioside A", "stevioside", and "rebaudioside C" are steviol glycosides, they may have structural characteristics of Formula 1 above. Specifically, the rebaudioside A may be represented by Formula 2 below, the stevioside may be represented by Formula 3 below, and the rebaudioside C may be represented by Formula 4 or 5 below.

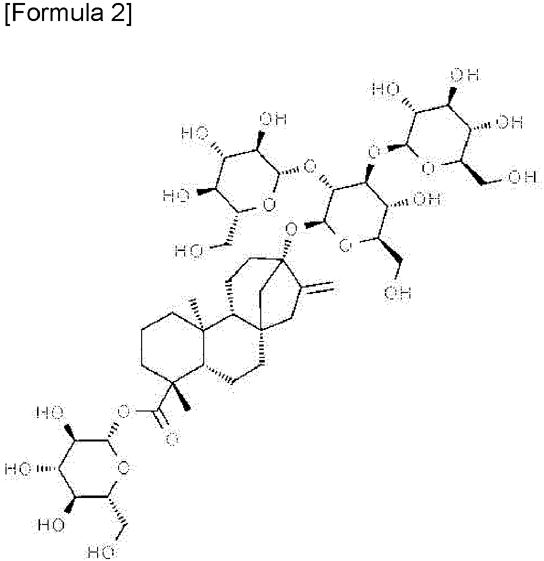

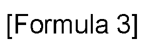

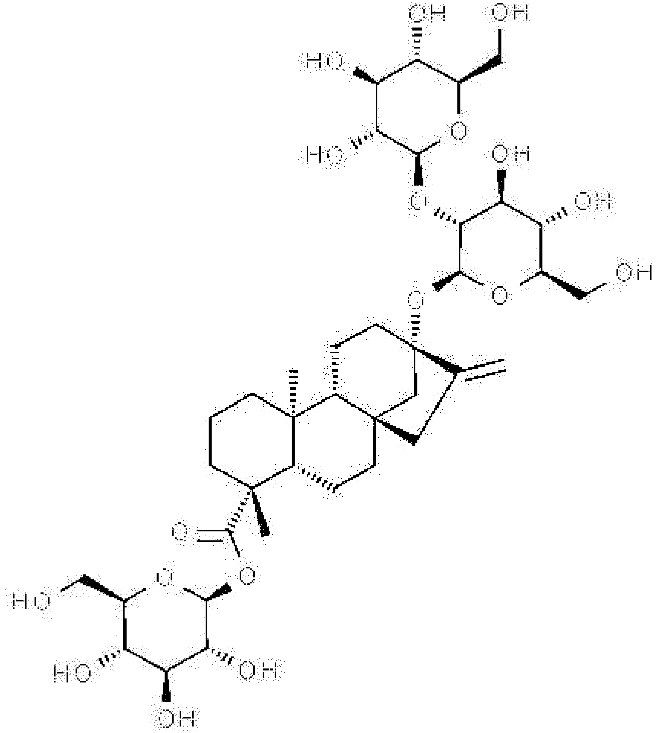

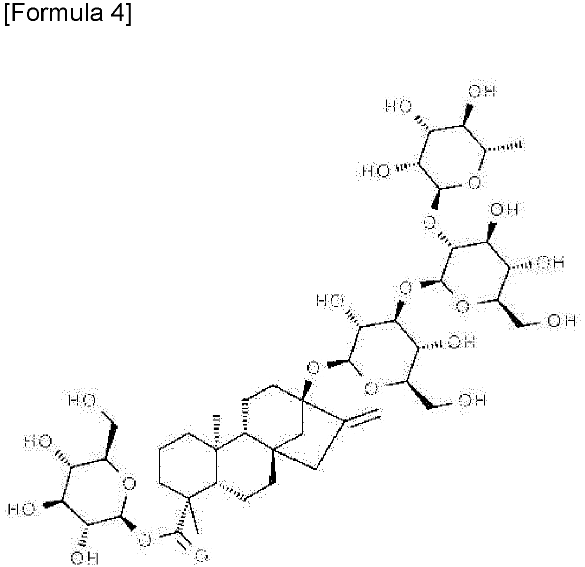

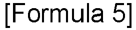

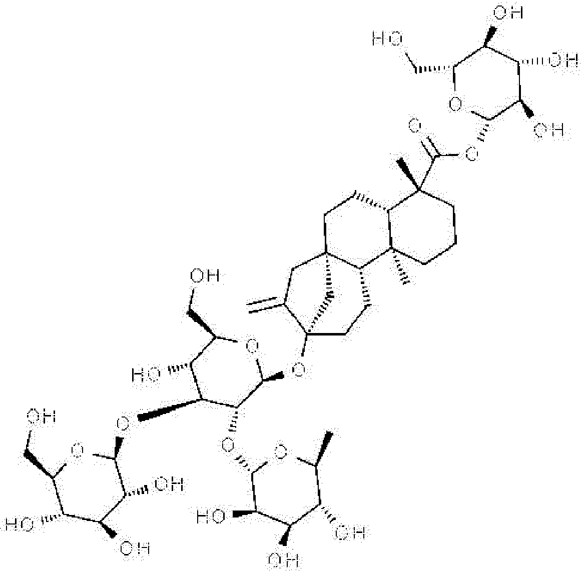

In the present disclosure, the "glucosylated steviol glycoside" may be a product in which glucose is added to a steviol glycoside.

The glucosylated steviol glycoside may be in a form in which 1 to 4 glucose molecules are added to a 19-OH site (-OH of C₁₉ in the case where R₁ of Formula 1 is H) via an α-bond, more specifically, in a form in which 1 to 4 glucose molecules are added to glucose conjugated to the 19-OH site of the steviol glycoside via an α-(1,6) bond, without being limited thereto.

The glucosylated rebaudioside A may be, for example, in a form in which glucose is added to a C₆ position of glucose conjugated to the C₁₉ site via an α-(1,6) bond and may be 13-[(2-*O*-β-D-glucopyranosyl-3-*O*-β-D-glucopyranosyl-β-D-glucopyranosyl)oxy]ent-kaur-16-en-19-oic acid 6-*O*-α-D-glucopyranose-β-D-glucopyranosyl ester as shown in Formula 6, but is not limited thereto.

The glucosylated stevioside may be, for example, in a form in which glucose is added to a C₆ position of glucose conjugated to the C₁₉ site via an α-(1,6) bond, more specifically, 13-[(2-*O*-β-D-glucopyranosyl-β-D-glucopyranosyl)oxy]ent-kaur-16-en-19-oic acid 6-*O*-α-D-glucopyranosyl-β-D-glucopyranosyl ester as shown in Formula 7, but is not limited thereto.

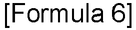

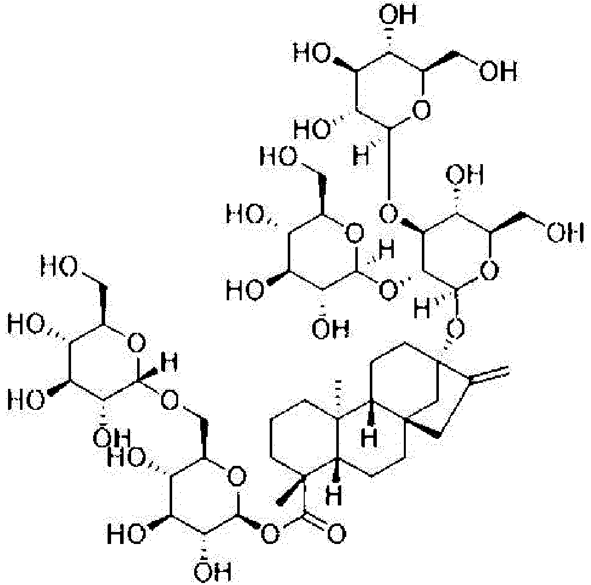

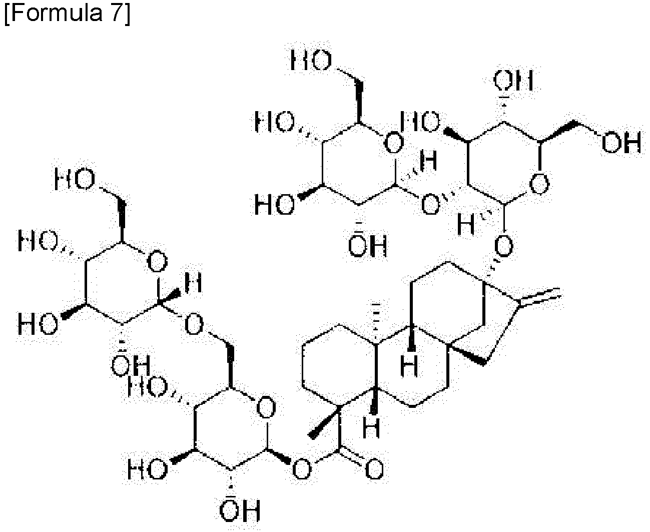

The glucosylated steviol glycoside may be prepared using sugar and the steviol glycoside as substrates by adding glucose to a steviol glycoside by transglucosylase, transglucosylase including an amino acid sequence of SEQ ID NO: 1, a microorganism expressing the same, or a culture of the microorganism or *Lactobacillus mali,* without being limited thereto.

The "glucosylated rebaudioside A" may be a product in which glucose is added to rebaudioside A, the "glucosylated stevioside" may be a product in which glucose is added to stevioside, and the "glucosylated rebaudioside C" may be a product in which glucose is added to rebaudioside C.

The glucosylated rebaudioside A, glucosylated stevioside, and glucosylated rebaudioside C may be products in which glucose is added through glucose conjugated to the 19-OH site via an α-(1,6) bond, without being limited thereto.

The glucosylated rebaudioside A, glucosylated stevioside, and glucosylated rebaudioside C may include 1 to 4 glucose molecules, without being limited thereto.

Specifically, the sweetener composition may include glucosylated rebaudioside A in an amount of 5 to 30 parts by weight, 6 to 30 parts by weight, 7 to 30 parts by weight, 8 to 30 parts by weight, 9 to 30 parts by weight, 10 to 30 parts by weight, 11 to 30 parts by weight, 11.33 to 30 parts by weight, 12 to 30 parts by weight, 13 to 30 parts by weight, 13.09 to 30 parts by weight, 14 to 30 parts by weight, 14.4 to 30 parts by weight, 5 to 28 parts by weight, 6 to 28 parts by weight, 7 to 28 parts by weight, 8 to 28 parts by weight, 9 to 28 parts by weight, 10 to 28 parts by weight, 11 to 28 parts by weight, 11.33 to 28 parts by weight, 12 to 28 parts by weight, 13 to 28 parts by weight, 13.09 to 28 parts by weight, 14 to 28 parts by weight, 14.4 to 28 parts by weight, 5 to 20 parts by weight, 6 to 20 parts by weight, 7 to 20 parts by weight, 8 to 20 parts by weight, 9 to 20 parts by weight, 10 to 20 parts by weight, 11 to 20 parts by weight, 11.33 to 20 parts by weight, 12 to 20 parts by weight, 13 to 20 parts by weight, 13.09 to 20 parts by weight, 14 to 20 parts by weight, 14.4 to 20 parts by weight, 5 to 15 parts by weight, 6 to 15 parts by weight, 7 to 15 parts by weight, 8 to 15 parts by weight, 9 to 15 parts by weight, 10 to 15 parts by weight, 5 to 15 parts by weight, 6 to 15 parts by weight, 7 to 15 parts by weight, 8 to 15 parts by weight, 9 to 15 parts by weight, 10 to 15 parts by weight, 11 to 15 parts by weight, 11.33 to 15 parts by weight, 12 to 15 parts by weight, 13 to 15 parts by weight, 13.09 to 15 parts by weight, 5 to 14.4 parts by weight, 6 to 14.4 parts by weight, 7 to 14.4 parts by weight, 8 to 14.4 parts by weight, 9 to 14.4 parts by weight, 10 to 14.4 parts by weight, 11 to 14.4 parts by weight, 11.33 to 14.4 parts by weight, 12 to 14.4 parts by weight, 13 to 14.4 parts by weight, 13.09 to 14.4 parts by weight, 5 to 13.09 parts by weight, 6 to 13.09 parts by weight, 7 to 13.09 parts by weight, 8 to 13.09 parts by weight, 9 to 13.09 parts by weight, 10 to 13.09 parts by weight, 11 to 13.09 parts by weight, or 11.33 to 13.09 parts by weight based on 100 parts by weight of the sweetener composition, without being limited thereto.

The sweetener composition may include glucosylated stevioside in an amount of 5 to 30 parts by weight, 6 to 30 parts by weight, 7 to 30 parts by weight, 8 to 30 parts by weight, 9 to 30 parts by weight, 10 to 30 parts by weight, 11 to 30 parts by weight, 11.41 to 30 parts by weight, 12 to 30 parts by weight, 13 to 30 parts by weight, 14 to 30 parts by weight, 15 to 30 parts by weight, 16 to 30 parts by weight, 17 to 30 parts by weight, 18 to 30 parts by weight, 19 to 30 parts by weight, 20 to 30 parts by weight, 20.12 to 30 parts by weight, 21 to 30 parts by weight, 22 to 30 parts by weight, 23 to 30 parts by weight, 24 to 30 parts by weight, 25 to 30 parts by weight, 26 to 30 parts by weight, 26.6 to 30 parts by weight, 5 to 27 parts by weight, 6 to 27 parts by weight, 7 to 27 parts by weight, 8 to 27 parts by weight, 9 to 27 parts by weight, 10 to 27 parts by weight, 11 to 27 parts by weight, 11.41 to 27 parts by weight, 12 to 27 parts by weight, 13 to 27 parts by weight, 14 to 27 parts by weight, 15 to 27 parts by weight, 16 to 27 parts by weight, 17 to 27 parts by weight, 18 to 27 parts by weight, 19 to 27 parts by weight, 20 to 27 parts by weight, 20.12 to 27 parts by weight, 5 to 26.6 parts by weight, 6 to 26.6 parts by weight, 7 to 26.6 parts by weight, 8 to 26.6 parts by weight, 9 to 26.6 parts by weight, 10 to 26.6 parts by weight, 11 to 26.6 parts by weight, 11.41 to 26.6 parts by weight, 12 to 26.6 parts by weight, 13 to 26.6 parts by weight, 14 to 26.6 parts by weight, 15 to 26.6 parts by weight, 16 to 26.6 parts by weight, 17 to 26.6 parts by weight, 18 to 26.6 parts by weight, 19 to 26.6 parts by weight, 20 to 26.6 parts by weight, 20.12 to 26.6 parts by weight, 5 to 25 parts by weight, 6 to 25 parts by weight, 7 to 25 parts by weight, 8 to 25 parts by weight, 9 to 25 parts by weight, 10 to 25 parts by weight, 11 to 25 parts by weight, 11.41 to 25 parts by weight, 12 to 25 parts by weight, 13 to 25 parts by weight, 14 to 25 parts by weight, 15 to 25 parts by weight, 16 to 25 parts by weight, 17 to 25 parts by weight, 18 to 25 parts by weight, 19 to 25 parts by weight, 20 to 25 parts by weight, 5 to 20.12 parts by weight, 6 to 20.12 parts by weight, 7 to 20.12 parts by weight, 8 to 20.12 parts by weight, 9 to 20.12 parts by weight, 10 to 20.12 parts by weight, 11 to 20.12 parts by weight, 11.41 to 20.12 parts by weight, 12 to 20.12 parts by weight, 13 to 20.12 parts by weight, 14 to 20.12 parts by weight, 15 to 20.12 parts by weight, 16 to 20.12 parts by weight, 17 to 20.12 parts by weight, 18 to 20.12 parts by weight, 19 to 20.12 parts by weight, or 20 to 20.12 parts by weight based on 100 parts by weight of the sweetener composition, without being limited thereto.

The sweetener composition may include glucosylated rebaudioside C in an amount of 5 to 20 parts by weight, 6 to 20 parts by weight, 7 to 20 parts by weight, 7.12 to 20 parts by weight, 8 to 20 parts by weight, 9 to 20 parts by weight, 9.26 to 20 parts by weight, 10 to 20 parts by weight, 10.24 to 20 parts by weight, 5 to 15 parts by weight, 6 to 15 parts by weight, 7 to 15 parts by weight, 7.12 to 15 parts by weight, 8 to 15 parts by weight, 9 to 15 parts by weight, 9.26 to 15 parts by weight, 10 to 15 parts by weight, 10.24 to 15 parts by weight, 5 to 11 parts by weight, 6 to 11 parts by weight, 7 to 11 parts by weight, 7.12 to 11 parts by weight, 8 to 11 parts by weight, 9 to 11 parts by weight, 9.26 to 11 parts by weight, 10 to 11 parts by weight, 10.24 to 11 parts by weight, 5 to 10.24 parts by weight, 6 to 10.24 parts by weight, 7 to 10.24 parts by weight, 7.12 to 10.24 parts by weight, 8 to 10.24 parts by weight, 9 to 10.24 parts by weight, 9.26 to 10.24 parts by weight, 10 to 10.24 parts by weight, 5 to 10 parts by weight, 6 to 10 parts by weight, 7 to 10 parts by weight, 7.12 to 10 parts by weight, 8 to 10 parts by weight, 9 to 10 parts by weight, 9.26 to 10 parts by weight, 5 to 9.26 parts by weight, 6 to 9.26 parts by weight, 7 to 9.26 parts by weight, 7.12 to 9.26 parts by weight, 8 to 9.26 parts by weight, or 9 to 9.26 parts by weight based on 100 parts by weight of the sweetener composition, without being limited thereto.

In a specific embodiment, the sweetener composition may further include glucosylated rebaudioside F, glucosylated dulcoside A, or glucosylated rubusoside, without being limited thereto.

In the present disclosure, "glucosylated rebaudioside F" is a type of glucosylated steviol glycoside in which glucose is added to "rebaudioside F".

In the present disclosure, "rebaudioside F", as a type of steviol glycoside, may have structural characteristics of Formula 1 and may be represented by Formula 8 below.

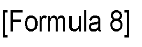

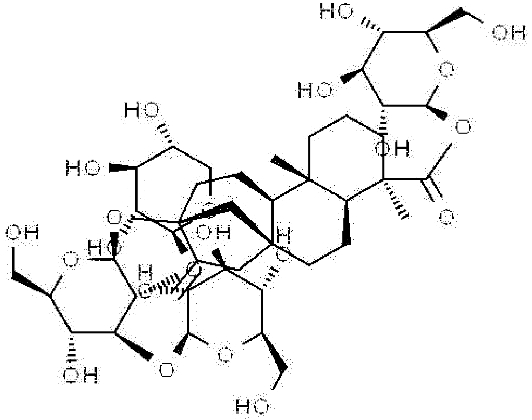

In the present disclosure, "glucosylated dulcoside A" is a type of glucosylated steviol glycoside in which glucose is added to "dulcoside A".

In the present disclosure, "dulcoside A", as a type of steviol glycoside, may have structural characteristics of Formula 1 and may be represented by Formula 9 below.

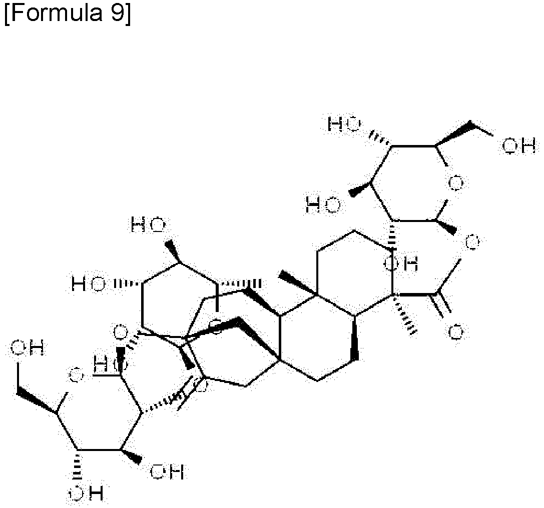

In the present disclosure, "glucosylated rubusoside" is a type of glucosylated steviol glycoside in which glucose is added to "rubusoside".

In the present disclosure, "rubusoside", as a type of steviol glycoside, may have structural characteristics of Formula 1 and may be represented by Formula 10 below.

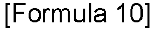

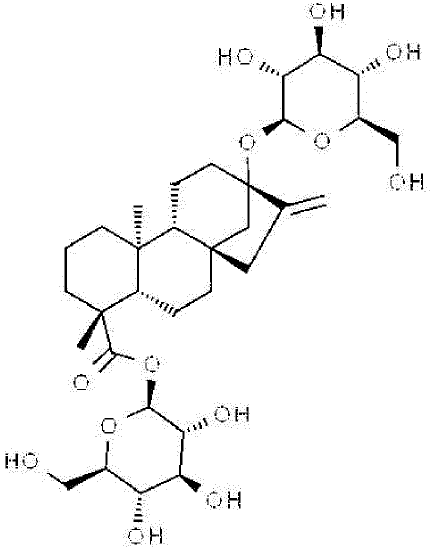

The sweetener composition may further include at least one selected from the group consisting of glucosylated rebaudioside F in an amount of 0.5 to 3 parts by weight, glucosylated dulcoside A in an amount of 0.5 to 3 parts by weight, and glucosylated rubusoside in an amount of 0.5 to 5 parts by weight based on 100 parts by weight of the sweetener composition, without being limited thereto.

Specifically, the sweetener composition may include glucosylated rebaudioside F in an amount of 0.5 to 3 parts by weight, 0.5 to 2 parts by weight, 0.5 to 1.5 parts by weight, 1 to 3 parts by weight, 1 to 2 parts by weight, or 1.03 to 1.5 parts by weight based on 100 parts by weight of the sweetener composition, without being limited thereto.

The sweetener composition may include glucosylated dulcoside A in an amount of 0.5 to 3 parts by weight, 0.5 to 2 parts by weight, 0.5 to 1.93 parts by weight, 0.5 to 1.23 parts by weight, 1 to 3 parts by weight, 1 to 2 parts by weight, 1 to 1.93 parts by weight, 1 to 1.23 parts by weight, 1.08 to 3 parts by weight, 1.08 to 1.93 parts by weight, 1.08 to 1.23 parts by weight, or 1.23 to 1.93 parts by weight based on 100 parts by weight of the sweetener composition, without being limited thereto.

The sweetener composition may include glucosylated rubusoside in an amount of 0.5 to 5 parts by weight, 0.5 to 4 parts by weight, 0.5 to 3 parts by weight, 0.5 to 2.42 parts by weight, 0.5 to 1.52 parts by weight, 1 to 5 parts by weight, 1 to 4 parts by weight, 1 to 3 parts by weight, 1 to 2.42 parts by weight, 1 to 1.52 parts by weight, 1.27 to 5 parts by weight, 1.27 to 4 parts by weight, 1.27 to 3 parts by weight, 1.27 to 2.42 parts by weight, 1.27 to 1.52 parts by weight, 1.52 to 5 parts by weight, 1.52 to 4 parts by weight, 1.52 to 3 parts by weight, or 1.52 to 2.42 parts by weight based on 100 parts by weight of the sweetener composition, without being limited thereto.

The "steviol glycoside" and "glucosylated steviol glycoside" are as described above.

The glucosylated steviol glycoside, glucosylated rebaudioside A, glucosylated stevioside, glucosylated rebaudioside C, or sweetener composition including the same may be prepared from a steviol glycoside by-product.

The glucosylated steviol glycoside, glucosylated rebaudioside A, glucosylated stevioside, glucosylated rebaudioside C, or sweetener composition may be prepared by using transglucosylase including an amino acid sequence of SEQ ID NO: 1, a microorganism expressing the same, or a culture of the microorganism or *Lactobacillus mali,* specifically by treating a steviol glycoside by-product generated in the preparation of stevia with transglucosylase including the amino acid sequence of SEQ ID NO: 1, a microorganism expressing the same, or a culture of the microorganism or *Lactobacillus mali,* without being limited thereto.

A method of using a culture broth of *Lactobacillus mali* is disclosed in Korean Patent Laid-open Publication No. 10-2019-0072471, which is incorporated herein by reference in its entirety. The culture broth of *Lactobacillus mali* may be used as a crude enzyme and may include transglucosylase including the amino acid sequence of SEQ ID NO: 1, and the amino acid sequence of SEQ ID NO: 1 may be derived from *Lactobacillus mali,* but is not limited thereto.

In the present disclosure, a "steviol glycoside by-product" may be a by-product generated during a stevia production process, and a type, composition ratio, content, and manufacturer thereof are not limited as long as the sweetener composition may be prepared. Specifically, the type of the steviol glycoside by-product is not particularly limited as long as the sweetener composition may be prepared via treatment with transglucosylase including the amino acid sequence of SEQ ID NO: 1 or a microorganism expressing the same or a culture of the microorganism or

### Lactobacillus mali.

Specifically, the steviol glycoside by-product may include at least one selected from the group consisting of stevioside, rubusoside, dulcoside A, rebaudioside A, rebaudioside C, rebaudioside D, rebaudioside E, rebaudioside F, and rebaudioside M, without being limited thereto.

The steviol glycoside by-product may be a liquid phase by-product in which crystallized rebaudioside A is isolated and may include rebaudioside A in an amount greater than 0 parts by weight and equal to or less than 30 parts by weight based on 100 parts by weight of the by-product, but is not limited thereto. The steviol glycoside by-product may include stevioside in an amount greater than 0 parts by weight and equal to or less than 45 parts by weight, greater than 0 parts by weight and equal to or less than 43.1 parts by weight, or greater than 0 parts by weight and equal to or less than 32 parts by weight based on 100 parts by weight of the by-product or include rebaudioside C in an amount greater than 0 parts by weight and equal to or less than 12 parts by weight or greater than 0 parts by weight and equal to or less than 11 parts by weight based on 100 parts by weight of the by-product, but is not limited thereto.

The steviol glycoside by-product may be a by-product generated in a stevia production process and may include rebaudioside A, stevioside, and rebaudioside C in a weight ratio of 1.5 to 2.5 : 1.5 to 6 : 1, without being limited thereto.

In the present disclosure, the "transglucosylase" refers to an enzyme that transfers glucose from a glucose donor to a glucose acceptor. Specifically, the transglucosylase may have a use for producing a glucosylated steviol glycoside by transferring glucose from a glucose donor to a steviol glycoside.

The transglucosylase may be an enzyme including the amino acid sequence of SEQ ID NO: 1. The transglucosylase including the amino acid sequence of SEQ ID NO: 1 may be an enzyme derived from a microorganism belonging to the genus *Lactobacillus,* specifically an enzyme derived from *Lactobacillus mali,* without being limited thereto.

A conversion rate of the transglucosylase including the amino acid sequence of SEQ ID NO: 1 from the steviol glycoside to the glucosylated steviol glycoside may be 50% or more, specifically 60% or more, more specifically 70% or more, even more specifically 80% or more, and most specifically 90% or more, without being limited thereto.

The transglucosylase including the amino acid sequence of SEQ ID NO: 1, the microorganism expressing the same, or the culture of the microorganism or *Lactobacillus mali* may degrade sugar into glucose and selectively link 1 to 4 glucose molecules to the steviol glycoside via an α-bond using sugar and the steviol glycoside as substrates, without being limited thereto.

The transglucosylase including the amino acid sequence of SEQ ID NO: 1, the microorganism expressing the same, or the culture of the microorganism or *Lactobacillus mali* may produce the sweetener composition including glucosylated rebaudioside A, glucosylated stevioside, and glucosylated rebaudioside C using sugar and the stevia by-product as substrates, wherein the sweetener composition may include 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener composition, but is not limited thereto.

In addition, the transglucosylase including the amino acid sequence of SEQ ID NO: 1, the microorganism expressing the same, or the culture of the microorganism or *Lactobacillus mali* may produce a sweetener composition further including glucosylated rebaudioside F using sugar and the stevia by-product as substrates, but is not limited thereto.

The enzyme including an amino acid sequence of SEQ ID NO: 1 may be used interchangeably with an enzyme having an amino acid sequence of SEQ ID NO: 1, an enzyme essentially consisting of an amino acid sequence of SEQ ID NO: 1, or an enzyme consisting of an amino acid sequence of SEQ ID NO: 1.

In the present disclosure, although transglucosylase is defined as an enzyme including the amino acid sequence of SEQ ID NO: 1, it will be obvious that any enzyme may be regarded as the transglucosylase of the present disclosure as long as the enzyme has an activity identical or equivalent to that of the enzyme having the amino acid sequence of SEQ ID NO: 1, essentially consisting of the amino acid sequence of SEQ ID NO: 1, or consisting of the amino acid sequence of SEQ ID NO: 1. For example, transglucosylase of the present disclosure may be a protein including the amino acid sequence of SEQ ID NO: 1 or consisting of an amino acid sequence having at least 80%, 90%, 95%, or 97% homology or identity with SEQ ID NO: 1.

That is, in the present disclosure, although the expression "enzyme, polypeptide, or protein including an amino acid sequence of a predetermined SEQ ID NO" or "enzyme, polypeptide, or protein consisting of an amino acid sequence of a predetermined SEQ ID NO" is described, it is obvious that any protein having an amino acid sequence including deletion, modification, substitution, or addition of one or several amino acids may be used in the present disclosure as long as the protein has an activity identical or equivalent to that of the polypeptide consisting of the amino acid sequence of the SEQ ID NO. For example, it is obvious that the "polypeptide consisting of the amino acid sequence of SEQ ID NO: 1" may belong to the "polypeptide consisting of amino acid sequence of SEQ ID NO: 1" as long as the activity is identical or equivalent.

As used herein, the term "homology" or "identity" refers to a degree of relevance between two given amino acid sequences or nucleotide sequences and may be expressed as a percentage. The terms homology and identity may often be used interchangeably.

Sequence homology or identity of conserved polynucleotides or polypeptides may be determined using a standard alignment algorithm and default gap penalties established by a program used together therewith. Substantially, homologous or identical sequences may hybridize with each other by at least about 50%, 60%, 70%, 80%, or 90% of the entire sequence or the entire length under moderate or highly stringent conditions. It is obvious that polynucleotides including a degenerate codon instead of a codon may also be considered in hybridization.

The homology, similarity, or identity between two polynucleotide or polypeptide sequences may be determined using any computer algorithm known in the art, e.g., "FASTA" program, using default parameters introduced by Pearson et al. (1988) Proc. Natl. Acad. Sci. USA 85:2444. Alternatively, the homology, similarity, or identity may be determined using the Needleman-Wunsch algorithm (Needleman and Wunsch, 1970, J. Mol. Biol. 48:443-453) as implemented in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST, from the National Center for Biotechnology Information database, or ClustalW.

The homology, similarity, or identity between polynucleotides or polypeptides may be determined by comparing sequence information using a GAP computer program as introduced by Needleman et al., (1970), J Mol Biol. 48:443 as disclosed by Smith and Waterman, Adv. Appl. Math (1981) 2:482. Briefly, the GAP program defines similarity as the number of aligned symbols (i.e., nucleotides or amino acids) which are similar, divided by the total number of symbols in a shorter of two sequences. Default parameters for the GAP program may include: (1) a binary comparison matrix (containing a value of 1 for identities and 0 for non identifies) and the weighted comparison matrix of Gribskov et al. (1986), Nucl. Acids Res. 14:6745 as described by Schwartz and Dayhoff, eds., Atlas Of Protein Sequence and Structure, National Biomedical Research Foundation, pp. 353-358 (1979) (or EDNAFULL (EMBOSS version of NCBI NUC4.4) substitution matrix); (2) a penalty of 3.0 for each gap and an additional penalty of 0.10 for each symbol in each gap (or a gap open penalty of 10 and a gap extension penalty of 0.5); and (3) no penalty for end gaps.

Another aspect of the present disclosure provides a composition for preparing a sweetener including a steviol glycoside by-product and transglucosylase including an amino acid sequence of SEQ ID NO: 1, a microorganism expressing the same, or a culture of the microorganism or *Lactobacillus mali.* Specifically, the sweetener includes 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener.

The composition for preparing a sweetener of the present disclosure may further include a cofactor capable of increasing glucose transfer efficiency to the steviol glycoside or improving stability of the sweetener composition, without being limited thereto. Examples of the cofactor may include a metal ion, a metal salt, an excipient, and a preservative, without being limited thereto.

The composition for preparing the sweetener may include transglucosylase including the amino acid sequence of SEQ ID NO: 1, a microorganism expressing the same, or a culture of the microorganism or *Lactobacillus mali,* without being limited thereto.

The "steviol glycoside by-product", "glucosylated rebaudioside A", "glucosylated stevioside", and "glucosylated rebaudioside C" are as described above.

Another aspect of the present disclosure provides a method of preparing a sweetener composition, the method including reacting a steviol glycoside by-product with transglucosylase including the amino acid sequence of SEQ ID NO: 1, a microorganism expressing the same, or a culture of the microorganism or *Lactobacillus mali* in the presence of a glucose donor. Specifically, the sweetener composition includes 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener composition.

In a specific embodiment, the steviol glycoside by-product may include at least one selected from the group consisting of stevioside, rubusoside, dulcoside A, rebaudioside A, rebaudioside C, rebaudioside D, rebaudioside E, rebaudioside F, and rebaudioside M, without being limited thereto.

The steviol glycoside by-product is a by-product generated during a stevia production process and may include rebaudioside A, stevioside, and rebaudioside C in a weight ratio of 1.5 to 2.5 : 1.5 to 6 : 1, without being limited thereto.

The "glucose donor", "steviol glycoside by-product", "glucosylated rebaudioside A", "glucosylated stevioside", "glucosylated rebaudioside C", and "sweetener composition" are as described above.

Another aspect of the present disclosure provides a method of improving sweetness quality, the method including reacting a steviol glycoside by-product with transglucosylase including an amino acid sequence of SEQ ID NO: 1, a microorganism expressing the same, or a culture of the microorganism or *Lactobacillus mali* in the presence of a glucose donor. Specifically, a sweetener having improved sweetness quality includes glucosylated rebaudioside A, glucosylated stevioside, and glucosylated rebaudioside C. The sweetener includes 5 to 30 parts by weight of glucosylated rebaudioside A, 5 to 30 parts by weight of glucosylated stevioside, and 7 to 20 parts by weight of glucosylated rebaudioside C based on 100 parts by weight of the sweetener.

The "glucose donor", "steviol glycoside by-product", "transglucosylase", "glucosylated rebaudioside A", "glucosylated stevioside", and "glucosylated rebaudioside C" are as described above.

An aspect of the present disclosure provides a use of steviol glycoside by-product and transglucosylase including an amino acid sequence of SEQ ID NO: 1, a microorganism expressing the same, or a culture of the microorganism or *Lactobacillus mali* for preparing a sweetener composition, wherein the sweetener composition comprises 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener composition.

The "steviol glycoside by-product", "transglucosylase", and "sweetener composition" are as described above.

An aspect of the present disclosure provides a use of steviol glycoside by-product and transglucosylase including an amino acid sequence of SEQ ID NO: 1, a microorganism expressing the same, or a culture of the microorganism or *Lactobacillus mali* for improving sweetness quality, wherein the sweetener comprises 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener.

The "steviol glycoside by-product", and "transglucosylase" are as described above.

### [Mode for Invention]

Hereinafter, the present disclosure will be described in more detail with reference to the following examples. However, the following examples are merely presented to exemplify the present disclosure, and the scope of the present disclosure is not limited thereto.

### Preparation Example 1: Preparation of Sweetener Composition Including Glucosylated Steviol Glycoside

A by-product of a steviol glycoside production process was obtained from a steviol glycoside manufacturer in China and used as a raw material for preparing a sweetener composition. Specifically, it was confirmed that the obtained by-product included rebaudioside A (Reb A), stevioside (STV), rebaudioside C (Reb C), and other steviol glycosides (rebaudioside F, dulcoside A, and rubusoside). In order to prepare a sweetener composition using the by-product, a culture broth of *Lactobacillus mali,* which is a lactic acid bacterium, was used as a crude enzyme liquid. A sweetener composition including glucosylated rebaudioside A, glucosylated stevioside, and glucosylated rebaudioside C was prepared using a method of adding glucose obtained from sugar to a steviol glycoside via a transglucosylating activity of the crude enzyme liquid. Structures of glucosylated rebaudioside A, glucosylated stevioside, and glucosylated rebaudioside C are illustrated in Formulae 2 to 7, respectively, and specific enzymatic reaction conditions are shown in Table 1 below.

A method of using a culture broth of *Lactobacillus mali* as a crude enzyme is disclosed in Korean Patent Laid-open Publication No. 10-2019-0072471, which is incorporated herein by reference in its entirety. The crude enzyme liquid contains transglucosylase including an amino acid sequence of SEQ ID NO: 1.

**Table 1**

| | |
|---|---|
| Concentration of raw material of steviol glycoside | 10% (w/v) |
| Weight ratio of steviol glycoside raw material: sugar | 1:1 |
| Reaction temperature | 40°C |
| Reaction pH | pH 5.0 |

High-Performance Liquid Chromatography (HPLC) was performed to analyze amounts of steviol glycosides and glucosylated steviol glycosides before and after the enzymatic reaction. The steviol glycosides subjected to HPLC analysis were rebaudioside A, stevioside, rebaudioside C, rubusoside, and dulcoside A, and the glucosylated steviol glycosides subjected to HPLC were glucosylated rebaudioside A, glucosylated stevioside, and glucosylated rebaudioside C. Analysis results of the HPLC are shown in FIGS. 1 to 3.

As a result, it was confirmed that the amounts of the components and conversion rates were as shown in Table 2 below and FIGS. 1 to 3 before the enzymatic reaction and 24 hours after the enzymatic reaction. It was also confirmed that the steviol glycosides such as rebaudioside A (Reb A), stevioside (STV), and rebaudioside C (Reb C) contained in the raw material were converted with a conversion rate of about 94% by transglucosylation via the enzymatic reaction to thereby prepare a sweetener composition including glucosylated rebaudioside A, glucosylated stevioside, and glucosylated rebaudioside C.

**Table 2**

| Sample | Components | Before enzymatic reaction (%) | 24 hours after enzymatic reaction (%) | Final conversion rate (%) |
|---|---|---|---|---|
| By-product of Company A | Rebaudioside A (Reb A) | 21.93 | 1.96 | 93 |
| | Stevioside (STV) | 35.56 | 1.71 | |
| | Rebaudioside C (Reb C) | 11.26 | 0.82 | |
| | Others (rebaudioside F (Reb F), dulcoside A, and rubusoside) | 5.83 | - | |
| By-product of Company B | Rebaudioside A (Reb A) | 22.23 | 1.96 | 93.62 |
| | Stevioside (STV) | 17.42 | 1.71 | |
| | Rebaudioside C (Reb C) | 11.60 | 0.82 | |
| | Others (rebaudioside F (Reb F), dulcoside A, and rubusoside) | 6.76 | 0.21 | |
| By-product of Company C | Rebaudioside A (Reb A) | 16.54 | 1.42 | 94.17 |
| | Stevioside (STV) | 45.84 | 2.20 | |
| | Rebaudioside C (Reb C) | 7.93 | 0.74 | |
| | Others (rebaudioside F (Reb F), dulcoside A, and rubusoside) | 4.42 | - | |

Meanwhile, types and contents of the steviol glycosides in the prepared composition 24 hours after the enzymatic reaction are shown in Tables 3 to 5 below. The composition prepared using the by-product of Company A is shown in Table 3, the composition prepared using the by-product of Company B is shown in Table 4 below, and the composition prepared using the by-product of Company C is shown in Table 5 below.

**Table 3**

| Type of steviol glycoside | Content (wt%) |
|---|---|
| Glucosylated rebaudioside A (Reb A-Glc) | 14.4 |
| Glucosylated stevioside (STV-Glc) | 20.12 |
| Glucosylated rebaudioside C (Reb C-Glc) | 9.26 |
| Glucosylated rebaudioside F | 1.26 |
| Glucosylated dulcoside A | 1.08 |
| Glucosylated rubusoside | 1.27 |
| Rebaudioside A (Reb A) | 1.96 |
| Stevioside (STV) | 1.71 |
| Rebaudioside C (Reb C) | 0.82 |

**Table 4**

| Type of steviol glycoside | Content (wt%) |
|---|---|
| Glucosylated rebaudioside A (Reb A-Glc) | 13.09 |
| Glucosylated stevioside (STV-Glc) | 11.41 |
| Glucosylated rebaudioside C (Reb C-Glc) | 10.24 |
| Glucosylated rebaudioside F | 1.5 |
| Glucosylated dulcoside A | 1.93 |
| Glucosylated rubusoside | 2.42 |
| Rebaudioside A (Reb A) | 1.91 |
| Stevioside (STV) | 0.72 |
| Rebaudioside C (Reb C) | 0.85 |

**Table 5**

| Type of steviol glycoside | Content (wt%) |
|---|---|
| Glucosylated rebaudioside A (Reb A-Glc) | 11.33 |
| Glucosylated stevioside (STV-Glc) | 26.6 |
| Glucosylated rebaudioside C (Reb C-Glc) | 7.12 |
| Glucosylated rebaudioside F | 1.03 |
| Glucosylated dulcoside A | 1.23 |
| Glucosylated rubusoside | 1.52 |
| Rebaudioside A (Reb A) | 1.42 |
| Stevioside (STV) | 2.2 |
| Rebaudioside C (Reb C) | 0.74 |

### Example 1: Sensory Test of Sweetener Composition

The sweetener compositions including the glucosylated steviol glycosides were evaluated in an organoleptic manner in comparison with high-purity rebaudioside A known in the art. Specifically, sensory preference tests were performed on the sweetener compositions of Tables 3 to 5 prepared in Preparation Example 1 above and commercially available rebaudioside A (97% purity), and the results were compared and evaluated. To this end, samples having the same sugar content of a Brix of 10° were prepared by adjusting the sugar content, and 30 people participated in taste testing. Preference, off-flavor, and off-odor were evaluated using a 9-point scale, and results converted to a 5-point scale are shown in Table 6 below.

**Table 6**

| Item of evaluation | Composition of Preparation Example 1 | Commercially available Reb A (97% purity) | P-Value |
|---|---|---|---|
| Preference for sweetener | 3.39 | 2.46 | 0.002 |
| Off-flavor/off-odor | 2.22 | 3.80 | 0.000005 |
| Overall preference | 3.56 | 1.85 | 0.0000002 |

As a result, it was confirmed that the sweetener composition including a glucosylated steviol glycoside having a certain composition ratio of Preparation Example 1 had increased sweetness preference by about 1.4-fold, reduced off-flavor/off-smell by 1/2, and increased overall preference by about 2-fold when compared with the rebaudioside A having a purity of 97%.

These results show that the glucosylated stevia composition having a certain composition ratio may be prepared in a one-pot process using the steviol glycoside production process with a culture broth of *Lactobacillus mali* as a crude enzyme liquid, and the glucosylated stevia composition having the certain composition may be used to prepare a high-value-added product with improved sweetness quality.

The above description of the present invention is provided for the purpose of illustration, and it would be understood by those skilled in the art that various changes and modifications may be made without changing the technical conception and essential features of the present invention. Thus, it is clear that the above-described embodiments are illustrative in all aspects and do not limit the present invention. Therefore, the scope of the disclosure is defined not by the detailed description, but by the claims and their equivalents, and all variations within the scope of the claims and their equivalents are to be construed as being included in the disclosure.

## Claims

1. A sweetener composition comprising glucosylated rebaudioside A, glucosylated stevioside, and glucosylated rebaudioside C,
wherein the sweetener composition comprises 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener composition.

2. The sweetener composition of claim 1, wherein the glucosylated rebaudioside A, glucosylated stevioside, and glucosylated rebaudioside C are in a form in which glucose is bound and added thereto through glucose conjugated to a 19-OH site via an α-(1,6) bond.

3. The sweetener composition of claim 2, wherein the glucosylated rebaudioside A, glucosylated stevioside, and glucosylated rebaudioside C comprise 1 to 4 glucose molecules.

4. The sweetener composition of claim 1, wherein the sweetener composition has improved sweetness quality compared to rebaudioside A having a purity of 95% or more.

5. The sweetener composition of claim 3, further comprising at least one selected from the group consisting of glucosylated rebaudioside F in an amount of 0.5 to 3 parts by weight, glucosylated dulcoside A in an amount of 0.5 to 3 parts by weight, and glucosylated rubusoside in an amount of 0.5 to 5 parts by weight based on 100 parts by weight of the sweetener composition.

6. A composition for preparing a sweetener, the composition comprising a steviol glycoside by-product and transglucosylase including an amino acid sequence of SEQ ID NO: 1, a microorganism expressing the same, or a culture of the microorganism or *Lactobacillus mali,*
wherein the sweetener comprises 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener.

7. A method of preparing a sweetener composition, the method comprising:
reacting a steviol glycoside by-product with transglucosylase including an amino acid sequence of SEQ ID NO: 1, a microorganism expressing the same, or a culture of the microorganism or *Lactobacillus mali,* in the presence of a glucose donor,
wherein the sweetener composition comprises 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener composition.

8. The method of claim 7, wherein the steviol glycoside by-product is a liquid-phase by-product in which crystalized rebaudioside A is isolated.

9. The method of claim 7, wherein the steviol glycoside by-product comprises rebaudioside A in amount greater than 0 parts by weight and equal to or less than 30 parts by weight based on 100 parts by weight of the by-product.

10. The method of claim 7, wherein the steviol glycoside by-product is a by-product generated in a stevia production process and comprises rebaudioside A, stevioside, and rebaudioside C in a weight ratio of 1.5 to 2.5 : 1.5 to 6 : 1.

11. The method of claim 7, wherein the glucose donor is sugar.

12. A method of improving sweetness quality, the method comprising:
reacting a steviol glycoside by-product with transglucosylase including an amino acid sequence of SEQ ID NO: 1, a microorganism expressing the same, or a culture of the microorganism or *Lactobacillus mali,* in the presence of a glucose donor,
wherein a sweetener having improved sweetness quality comprises glucosylated rebaudioside A, glucosylated stevioside, and glucosylated rebaudioside C,
wherein the sweetener comprises 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener.

13. A use of steviol glycoside by-product and transglucosylase including an amino acid sequence of SEQ ID NO: 1, a microorganism expressing the same, or a culture of the microorganism or *Lactobacillus mali* for preparing a sweetener composition.
wherein the sweetener composition comprises 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener composition.

14. A use of steviol glycoside by-product and transglucosylase including an amino acid sequence of SEQ ID NO: 1, a microorganism expressing the same, or a culture of the microorganism or *Lactobacillus mali* for improving sweetness quality.
wherein the sweetener comprises 5 to 30 parts by weight of glucosylated rebaudioside A, 5 to 30 parts by weight of glucosylated stevioside, and 5 to 20 parts by weight of glucosylated rebaudioside C based on 100 parts by weight of the sweetener.
